(19) Europäisches Patentamt

European Patent Office

Office européen des brevets

(11) **EP 4 280 218 A1**

(12) **EUROPEAN PATENT APPLICATION**

(43) Date of publication:
**22.11.2023 Bulletin 2023/47**

(21) Application number: **22305728.2**

(22) Date of filing: **17.05.2022**

(51) International Patent Classification (IPC):
**G16B 20/00** (2019.01)       **G16B 25/10** (2019.01)
**G16B 40/30** (2019.01)

(52) Cooperative Patent Classification (CPC):
**G16B 25/10; G16B 20/00; G16B 40/30**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(71) Applicant: **PIERRE FABRE MEDICAMENT**
**81500 Lavaur (FR)**

(72) Inventor: **BONNET, Roland**
**81490 Noailhac (FR)**

(74) Representative: **Regimbeau**
**20, rue de Chazelles**
**75847 Paris Cedex 17 (FR)**

(54) **METHOD OF TRANSCRIPTOMIC ANALYSIS OF A BIOLOGICAL SAMPLE**

(57)    The present invention relates to a method for training an autoencoder-type neural network for transcriptomic analysis of a biological sample comprising the implementation of steps of:

(a0) for each training gene expression vector of a batch of training gene expression vectors representing at least a part of a transcriptome of a cell,
- projecting the training gene expression vector into an embedding space of reduced dimension with an encoder block of the autoencoder;
- reconstructing an approximation of the training gene expression vector from its projection into said embedding space;

(a1) applying a clustering algorithm to the projections of the training gene expression vectors of said batch;
(a2) for a plurality of pairs of training gene expression vectors of said batch, predicting with a discriminator neural network whether they are assigned to the same cluster;
(a3) minimizing a loss function comprising at least:
- a first term representative of a reconstruction error of the autoencoder;
- a second term representative of a cluster segregation error of the autoencoder, as a function of the predictions of the discriminator.

A method for transcriptomic analysis of a biological sample is further proposed.

FIG. 1a

FIG. 1b

## Description

FIELD OF THE INVENTION

The field of this invention is that of deep learning.

More particularly, the invention relates to methods for training an autoencoder-type neural network, and for transcriptomic analysis of a biological sample.

BACKGROUND OF THE INVENTION

*RNAseq*

Ribonucleic acid (RNA) has multiple forms and plays a critical role in cell growth and differentiation. RNA transcription and stability are tightly regulated in response to physiological and pathological stimuli. Abnormal expression of RNA is frequently associated with human cancer initiation, development, progression and metastasis.

In addition to the mutation of tumor suppressor genes and oncogenes, gene expression could be overactivated or epigenetically silenced which could lead to uncontrolled tumor cell growth and proliferation. Aberrant activation of cell growth signaling pathways and/or transcription factors could lead to high-level expression of genes associated with tumor development and progression. Different gene expression profiles may reflect different cancer subtypes, the stage of cancer development or tumor microenvironment.

Ribonucleic acid sequencing (RNAseq) allows the identification of all the transcripts in a sample, replacing other methods such as RT-PCR / RT-qPCR and Microarrays. While RT-PCR / RT-qPCR measures the expression level of one known transcript, Microarrays offer the possibility of covering a wide range of targets. However, both of them measure the expression level of predetermined transcripts, therein limiting the discovery of new processes. On the other hand, RNAseq is able to sequence the whole transcriptome, enabling the sequencing of non-model organisms whose genome is unknown. RNAseq can reveal gene fusions, splicing variants, mutations/indels in addition to differential gene expression, thus providing a more complete genetic picture than DNA sequencing. Therefore, RNAseq is a powerful tool for understanding the molecular mechanisms of cancer development and developing novel strategies for cancer prevention and treatment.

*Bulk RNAseq*

Since the first Expressed Sequence Tag (EST) library was sequenced using LCM-454 sequencer in 2007, bulk RNA seq has become the most valuable and extensively used tool in understanding cancer biology.

Bulk RNAseq has broad utilities in cancer classification, biomarker and gene fusion discoveries, disease diagnosis, and optimizing therapeutic treatment. The translational research targeted for clinical oncology is primarily in two areas. The most informative area is the biomarker discoveries for cancer diagnosis, prognosis, and prediction. Numerous RNAseq-based signatures have been developed and validated across many major tumor types.

Using bulk RNAseq, numerous novel gene fusions have been discovered, many of which can be directly beneficial as FDA approved drugs or offer new therapeutic opportunities.

Large-scale RNAseq experiments not only brought a lot of new findings to the field of non-coding RNA but also to transcriptomics by improving genome annotations. These revealed that the transcribed genome regions are wider than previously expected. Bulk RNAseq includes diverse types of RNA species and detects multiple forms of genomic alterations at a single base resolution, many of which are not detected by the DNA approach or other traditional methods.

However, like for other bulk experiments, bulk RNAseq can have some limitations for heterogeneous samples, including organs, biopsies, or during dynamic processes such as development and differentiation. The quantity of starting material needed for bulk RNAseq can also be an issue. Bulk RNAseq uses a tissue or cell population as a starting material, and results in a mixture of different gene expression profiles from the studied material. The transcriptome programs of tumors are highly heterogeneous both between tumor cells, due to somatic genetic alterations, and within tumor microenvironments, resulting from significant infiltration of the stroma and other cell types in the tumor. The true signals driving the tumorigenesis or therapeutic resistance from a rare cell population or cell type can be obscured by an average gene expression profile from bulk RNAseq.

*scRNAseq*

RNAseq technology, most widely used in genomics tool box, has evolved from classic bulk RNA sequencing (bulk RNAseq) to single cell RNA sequencing (scRNAseq). Bulk RNAseq is very informative and can lead to the identification of the molecular mechanisms underlying physiological or pathological processes. However, for researchers working with heterogeneous samples, including biopsies, bulk RNAseq can be limited. In order to overcome this limitation, single-cell technologies have emerged and have been adapted to different omic protocols such as scRNAseq. Bulk RNAseq studies average global gene expression whereas scRNAseq investigates single cell RNA biology up to 20,000 individual cells simultaneously. This biological challenge catalyzed the birth of scRNAseq, an alternative RNAseq technology.

Since 2017, scRNAseq has become one of the most popular genomic tools to dissect transcriptome heterogeneity, discovering rare cell types and cell states in tumors.

scRNAseq is a very powerful method that allows transcriptomic analysis of heterogeneous tissue, or dynamic processes in one single experiment. Depending on the number of cells and the sequencing depth, scRNAseq can be very sensitive.

scRNAseq is ideal for examination of single cells where each one is essentially unique (for example, individual T lymphocytes expressing highly diverse T-cell receptors). In addition to resolving cell heterogeneity, scRNAseq can also provide important information about fundamental characteristics of gene expression. scRNAseq is much better suited for biomedical research and for clinical applications. For example, the ability to study thousands of cells in a single run has greatly facilitated prospective studies of highly heterogeneous clinical samples. Whereas bulk RNA-Seq is quite straightforward to analyze the transcriptome, scRNAseq gives more information about the variations of the transcriptome during evolving processes (healthy or pathologic). In 2020, lot of research articles used scRNAseq technology, suggesting that single-cell technology is becoming widely adopted in research labs. Single-cell technology has paved the way to the discovery of under-representative cell populations, signaling pathways, pathological mechanisms, and among other for the identification of interest targets in immuno-oncology.

*Bioinformatic analysis*

Lots of all-inclusive solutions exist to analyze scRNAseq data. These include several Quality Controls (QC) and the interpretation of scRNAseq data. Companies selling reagents for single-cell experiments, usually also offer software to analyze the sequencing data (10X Genomics Loupe or Fluidigm Singular). QC mainly results in the exclusion of single-cells with poor-quality data. Once the data are filtered, the goal is to align the sequence to the genome, analyze the expression of every transcript across each cell, and cluster them. Different bioinformatics tools (algorithms and computational approaches) are available but they only provide a generic and non-discriminatory analysis. All conventional methods aggregate data and do not allow a good cell type separation. For example, these methods will allow to differentiate T lymphocytes (LT) but not their subclass (Naive T cells, T helpers, LT CD4, LT CD8 ...).

For instance, a PCA can be used to project the expression matrix space into an embedding space referred to as latent space (with a dimension of around 100), followed by Louvain algorithm for identifying clusters. Annotation can be done at the cluster level based on a database of cells profiles. See also the document TISCH: a comprehensive web resource enabling interactive single-cell transcriptome visualization of tumor microenvironment, by Dongqing Sun, Jin Wang, Ya Han, Xin Dong, Jun Ge, Rongbin Zheng, Xiaoying Shi, Binbin Wang, Ziyi Li, Pengfei Ren, Liangdong Sun, Yilv Yan, Peng Zhang, Fan Zhang, Taiwen Li, Chenfei Wang, in Nucleic Acids Research, Volume 49, Issue D1, 8 January 2021, Pages D1420-D1430, https://doi.org/10.1093/nar/gkaa1020.

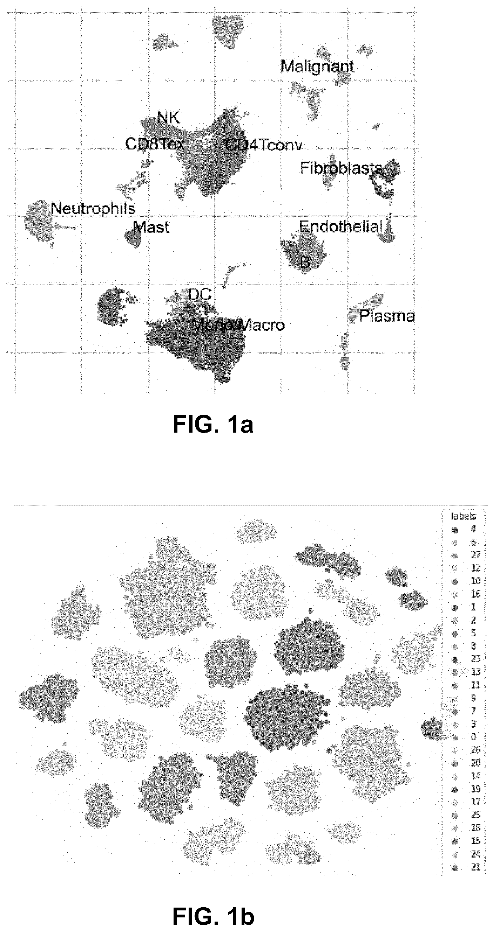

As depicted by **Figure 1a,** the annotation is correct, but poor cell segregation is unfortunately observed. In the example of figure 1a, lymphoids are neatly separated from myeloids, but subtypes of T (and NK) are poorly separated and sometimes merged. It induces errors in subtype phenotype, and lack of confidence.

The objective to the present invention is to allow, for each cell, to determine the expression of identified targets and biomarkers using an improved treatment algorithm. In other words to know in which cell type the target or the biomarker is expressed, depending on the disease, the disorder, or the inflammation, for example a cancer indication. The invention consists in determining the best separators (canonical genes) assuming that there are subgroups in the data.

The advantages of the invention are the followings:

- improving the clustering of the cell in a specific disease or disorder (e.g. cancer, inflammatory disease, inflammatory disorder, etc.);
- improving the separation of the cluster for a better identification of cell types (the sorting of cell is improved by labelling cell with a new algorithm), and
- having a good cell segregation (higher resolution and limited background noise and separation between the cluster (improve the quality of the response), such as depicted by **figure 1b**.

The algorithm enables to maximize the gap between the clusters for obtaining a better clustering. The invention then allows, from the transcriptomic analysis of a biological sample, the selection of patient stratification according tumor microenvironment setting (with certain targets and cells of interest), the determination of pathway, the establishment of the prevalence analysis of a target, and find new targets and biomarkers by ranking genes among specific cell types (oncogenes, immune checkpoints, etc.).

SUMMARY OF THE INVENTION

For these purposes, the present invention provides according to a first aspect a method for training an autoencoder-type neural network for transcriptomic analysis of a biological sample, the method being characterized in that it comprises the implementation, by a data processor of a first server, for at least a batch of training gene expression vectors from a database, each training gene expression vector representing at least a part of a transcriptome of a cell, of steps of:

(a0) for each training gene expression vector of said batch,

- projecting the training gene expression vector into an embedding space of a given reduced dimension with respect to the dimension of the gene expression vector, with an encoder block of the autoencoder-type neural network;
- reconstructing an approximation of the training gene expression vector from its projection into said embedding space, with an encoder block of the autoencoder-type neural network;

(a1) applying a clustering algorithm to the projections into said embedding space of the training gene expression vectors of said batch so that each training gene expression vector of the batch is assigned to a cluster among a plurality of clusters;

(a2) for a plurality of pairs of training gene expression vectors of said batch, predicting with a discriminator neural network whether they are assigned to the same cluster;

(a3) modifying parameters of the autoencoder-type neural network and the discriminator neural network so as to minimize a loss function comprising at least:

- a first term representative of a reconstruction error of the autoencoder-type neural network, as a function of the training gene expression vectors and the reconstructed approximations of said training gene expression vectors;
- a second term representative of a cluster segregation error of the autoencoder-type neural network, as a function of the predictions of the discriminator.

Preferred but non limiting features of the present invention are as follow:
Said first term is the Mean-Square error between the training gene expression vectors and the reconstructed approximations of said training gene expression vectors.

Said second term tends toward 0 if the discriminator neural network correctly predicts whether a pair of training gene expression vector are assigned to the same cluster, and tends toward 1 if the discriminator neural network incorrectly predicts whether a pair of training gene expression vectors are assigned to the same cluster.

The discriminator neural network outputs a predicted probability score that a pair of training gene expression vectors are assigned to the same cluster, said second term being computed as the Binary Cross Entropy between said predicted probability score and a ground truth equal to 1 if the pair of training gene expression vectors are actually assigned to the same cluster, and equal to 0 if the pair of training gene expression vectors are actually not assigned to the same cluster.

Said loss function is given by the formula *MSE* + $\lambda$*BCE,* wherein MSE is the first term, *BCE* is the second term, and $\lambda$ a scalar with $0 < \lambda \leq 1$.

Each of the encoder block and the decoder block comprises two sequences of a core layer and a non-linear activation layer, wherein the core layer is chosen among a fully connected layer, an attention layer and a convolution layer.

Both the first core layer of the encoder and the first core layer of the decoder comprise 256 nodes, and the second core layer of the encoder comprises 64 nodes, so that said embedding space is of dimension 64.

The gene expression vectors are of dimension at least 10000, in particular at least 20000.

Said clustering algorithm is the k-means algorithm.

According to a second aspect, the invention proposes a method for transcriptomic analysis of a biological sample, the method being characterized in that it comprises the implementation, by a data processor of a second server, of steps of:

(b) Obtaining from a client equipment, for a plurality of individual cells of said biological sample, a candidate gene expression vector representing at least a part of a transcriptome of the cell;

(c) for each candidate gene expression vector of the set of said candidate gene expression vectors, projecting the candidate gene expression vector into an embedding space of a given reduced dimension with respect to the dimension of the gene expression vector, with an encoder block of an autoencoder-type neural network obtainable by the method according to the first aspect;

(d) applying said clustering algorithm to the projections into said embedding space of the candidate gene expression vectors of said set so that each candidate gene expression vector of the set is assigned to a cluster among a plurality of clusters.

Preferred but non limiting features of the present invention are as follow:
The method comprises comprising a previous step (a) of training said autoencoder-type neural network according to the first aspect.

The method comprises a further step (e) of annotating each cluster with a cell type as a function of the most differentially expressed genes in candidate gene expression vectors of said cluster.

The method comprises a further step (f) of identifying a cluster matching a target signature gene expression in candidate gene expression vectors of said cluster.

According to a third and fourth aspects, the invention proposes a computer program product comprising code instructions to execute a method according to the first aspect for training an autoencoder-type neural network, or according to the second aspect for transcriptomic analysis of a biological sample; and a computer-readable medium, on which is stored a computer program product comprising code instructions for executing a method according to the first aspect for training an autoencoder-type neural network, or according to the second aspect for transcriptomic analysis of a biological sample.

BRIEF DESCRIPTION OF THE DRAWINGS

The above and other objects, features and advantages of this invention will be apparent in the following detailed description of an illustrative embodiment thereof, which is to be read in connection with the accompanying drawings wherein:

- Figure 1a and 1b respectively illustrate a poor cluster segregation and a clean cluster segregation;
- figure 2 illustrates an example of architecture in which the method according to the invention is performed;
- figure 3 represents steps of an embodiment of a method for training an autoencoder-type neural network for transcriptomic analysis of a biological sampleaccording to the first aspect of the invention;
- figure 4 schematically illustrates the training strategy of the present invention;
- figure 5 represents steps of an embodiment of a method for transcriptomic analysis of a biological sampleaccording to the second aspect of the invention.

DETAILED DESCRIPTION OF A PREFERRED EMBODIMENT

*Definitions*

As used herein, "target" refers to a gene or gene product that is modulated, inhibited, or silenced by an agent, a composition, and/or a formulation or a physiological or pathological status. In immuno-oncology, a "target" means a cell surface or secreted molecule that can modulate tumor growth and/or the immunological response to a tumor. A target gene or gene product includes wild-type and mutant forms. Similarly, the term "target", "targets", or "targeting" used as a verb refers to modulating the activity of a target gene or gene product. Targeting may refer to upregulating or downregulating the activity of a target gene or gene product.

"Targets" also include, but are not limited to CTLA-4, PD-L1, PD-L2, PD1, B7-H3, B7-H4, BTLA, CD80, CD86, HVEM, ILT3, ILT4, TIGIT, VSIG3, VSIG4, CD160, CD40, ICOS, CD70, OX40, GITR, TIM3, GAL9, CD27, CD28, GITR, LAG3, PSG-L1, VISTA, KIR, IDO1, A2aR, ...

As used herein, "cell type" refers to cells defined by expression of a unique combination of genes. A classification is used to identify cells that share morphological or phenotypical features.

"Cell type" also include, but are not limited to B cells, proliferative B cells, plasma B cells, naive T cells, regulatory T cells, T helper cells, Th17 cells, proliferative T cells, CD4 T cells, CD8 T cells, Natural Killer, TAM (tumor associated macrophage), monocytes, macrophages, mastocytes cells, dendritic cells...

As used herein, "biomarker" refers to a cytogenetic marker, a molecule, a substance, the expression of which in a sample from a patient can be detected by standard methods in the art, and is predictive or denotes a condition of the biological sample from which it was obtained. The "biomarker" can indicate a disease state.

"Biomarker" includes polypeptides and glycoproteins, the quantities of which increase or decrease in patients suffering from a disease or prone to be afflicted by a disease as compared to normal healthy subjects.

As used herein, "collections of biological samples" or "biological samples" also include, but are not limited to tissues and its derivatives, cells, organs, blood and its components.

As used herein, "cancer indication" refers to the different types of cancer. "cancer" as used herein is any malignant neoplasm resulting from the undesired growth, the invasion, and under certain conditions metastasis of impaired cells in an organism. The cells giving rise to cancer are genetically impaired and have usually lost their ability to control cell division, cell migration behaviour, differentiation status and/or cell death machinery.

Cancer indications also include, but are not limited to, oesophageal carcinoma, melanoma cancer, colon cancer, hepatocellular carcinoma, breast cancer, triple negative breast cancer, breast ER+, breast HER2+, lung adenocarcinoma, squamous cell lung, glioma, gastrointestinal cancer, head and neck cancer, cholangiocarcinoma, kidney clear cell cancer, pancreatic cancer, osteosarcoma, uveal melanoma cancer, gastric cancer, salivary gland cancer, sarcoma, prostate cancer, glioblastoma, ovarian cancer, thyroid cancer, basal cell carcinoma, bladder cancer, endometrial cancer, chromophobe renal cancer, renal cell carcinoma, vaginal cancer, kidney papillary cancer, neuroendocrine carcinoma, peritoneum cancer, laryngeal cancer, liver cancer, kidney cancer, biliary tract cancer, stomach cancer, colorectal cancer, nasopharyngeal cancer, larynx cancer, mesothelioma cancer, lung cancer, cervical cancer, oesophageal cancer, oesophageal cervical cancer, haematological cancer (e.g., leukaemia, lymphomas, or myelomas) etc.

*Architecture*

According to two complementary aspects of the present invention are proposed:

- a method for training an autoencoder-type neural network for transcriptomic analysis of a biological sample;
- a method for transcriptomic analysis of a biological sample, using an autoencoder-type neural network, advantageously trained according to the first method.

By "transcriptomic analysis of a biological sample", it is meant processing a set of gene expression vectors ("expression profiles") representing at least a part of a transcriptome (set of all RNA transcripts) of a plurality of individual cells of said biological sample. To rephrase, each gene expression vector of said set gene expression corresponds to an individual cell of said biological sample, hence the name "Single-cell RNA-Seq" (scRNA-Seq). Each value of a gene expression vector quantifies the expression of one gene. Typically, the gene expression vectors are of dimension at least 10000 (i.e. 10000 genes are monitored), in particular at least 20000. Furthermore, said set advantageously comprises at least 1000 gene expression vectors (i.e. 1000 individual cells of said biological sampleare monitored), in particular at least 10000. The whole set of gene expression vectors can be represented by an "expression matrix" wherein each line corresponds to a gene and each column corresponds to an individual cell of the biological sample.

Note that cells with too few genes (in particular fewer than 200 genes) and/or a too small cell count (in particular fewer than 500 counts per cell) are preferably removed. The various gene expression vectors may also undergo normalization (typically "LogNormalize").

Said processing aims at "aggregating" the gene expression vectors of the set, in particular in order to reveal heterogeneity and subpopulation expression variability within the biological sample.

Therefore, this processing at least implies a projection of said set of gene expression vectors into an embedding space of a given lower dimension with respect to the dimension of the gene expression vector (said gene expression vector's dimension is preferably at least 100 times the embedding space's dimension, i.e. said dimension of the embedding space is less than 100, preferably 64), preferably an assignation of each gene expression vector to a cluster among a plurality of clusters in said embedding space, each cluster being very preferably annotated with a cell type, see below.

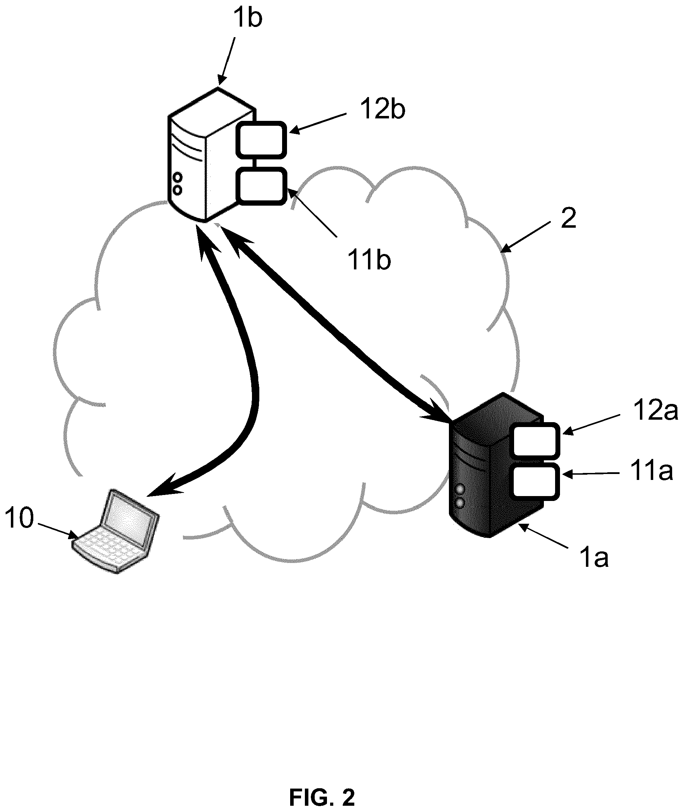

The above mentioned two types of methods are implemented within an architecture such as illustrated in **Figure 2,** by means of a first and/or second server 1a, 1b. The first server 1a is the learning server (implementing the first method) and the second server 1b is an analysis server (implementing the second method). It is fully possible that these two servers may be merged.

Each of these servers 1a, 1b is typically remote computer equipment connected to an extended network 2 such as the Internet for data exchange. Each one comprises data processing means 11a, 11b of processor type (in particular the data processing means 11a of the first server have strong computing power, since learning is long and complex compared with ordinary use of the trained autoencoder-type NN), and optionally storage means 12a, 12b such as a computer memory e.g. a hard disk.

The memory 12a of the first server 1a stores a training database i.e. a set of reference data which are already analyzed (as opposed to so-called inputted data that precisely is sought to be analyzed). As explained, the data is here constituted of gene expression vectors, the training examples being referred to as training gene expression vectors, preferably already annotated, i.e. each training gene expression vector is associated to the corresponding cell type as ground truth.

Several public training databases are known to the skilled person, for instance Tisch, GEO (Gene Expression Omnibus) or ArrayExpress.

The architecture advantageously comprises one or more items of client equipment 10, which may be any work station (also connected to network 2), preferably separate from the servers 1a, 1b but possibly being merged with one and/or the other thereof. The client equipment 10 acts as input device. Note that the set of gene expression vectors can be obtained from a sample of said biological sampleusing any technique known from the skilled person, and in particular the client 10 may comprise RNA/cDNA amplification and/or sequencing means. Alternatively, said client equipment 10 may be able to directly be provided with the data.

*Training method*

In a first aspect, there is proposed a training method, implemented by the data processing means 11a of the first server 1a. Said method trains an autoencoder-type neural network for transcriptomic analysis of a biological sample.

An autoencoder, or "encoder-decoder" is a neural network comprising two main parts: an encoder block that maps the input into a code (a high level feature map descriptive of its input), and a decoder block that maps the code back to its input. In order words, an autoencoder reconstructs an approximation of its input. The code space generally presents a dimension lower than the dimension of the input space, hence the capacity of an autoencoder to "denoise" its input.

The autoencoder here takes as input a gene expression vector, and the code space is used as the embedding space. In other words, an inputted gene expression vector input is projected by the encoder block of the autoencoder into the embedding space.

This allow reducing the dimension of the gene expression vector from above 10000 to less than 100 (preferably 64). As it will be seen, if properly trained in accordance with the present invention, an autoencoder allows making the embedding space separable enough to label clusters.

In a preferred embodiment, each of the encoder block and the decoder block comprises two sequences of a core layer (chosen among a fully-connected or "dense" layer, an attention layer, and a convolution layer, but preferably a fully-connected layer) and a non-linear activation layer (in particular ReLU). Note that there could be more than two sequences, and further layers such as batch normalization layers.

In this preferred embodiment, both the first core layer of the encoder and the first code layer of the decoder comprise N1 nodes, and the second core layer of the encoder comprises N2 nodes, so that said given dimension of the embedding space is N2, with $2*N2 \leq N1 \leq 16*N2$, very preferably $N1 = 4*N2$ (i.e. 256 if N2=64 as explained).

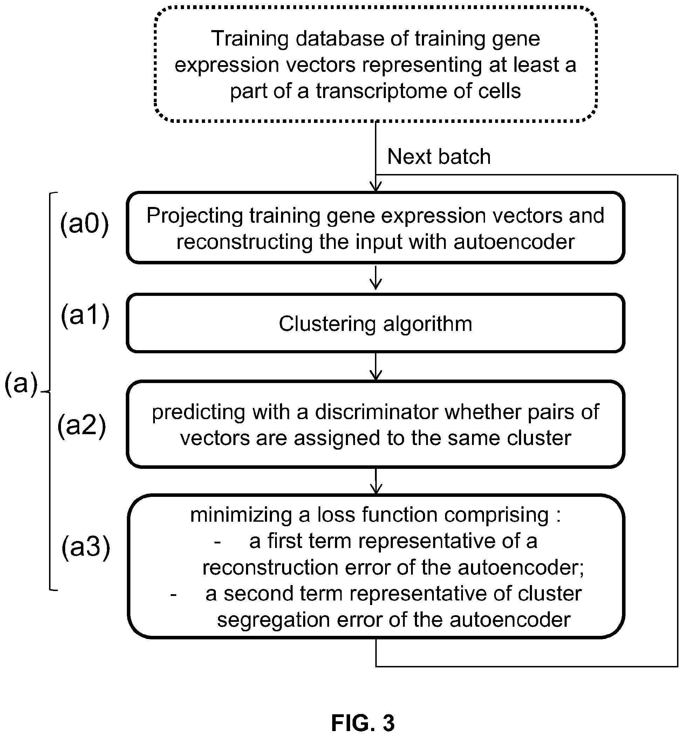

The present training is performed for at least a batch of training gene expression vectors from said database. Preferably a plurality of successive batches. In a first step (a0), illustrated by **figure 3,** for each training gene expression vector of said batch:

- the training gene expression vector is projected into said embedding space of the given reduced dimension with respect to the dimension of the gene expression vector, with the encoder block of the autoencoder-type neural network;
- an approximation of the training gene expression vector is reconstructed from its projection into said embedding space, with the encoder block of the autoencoder-type neural network.

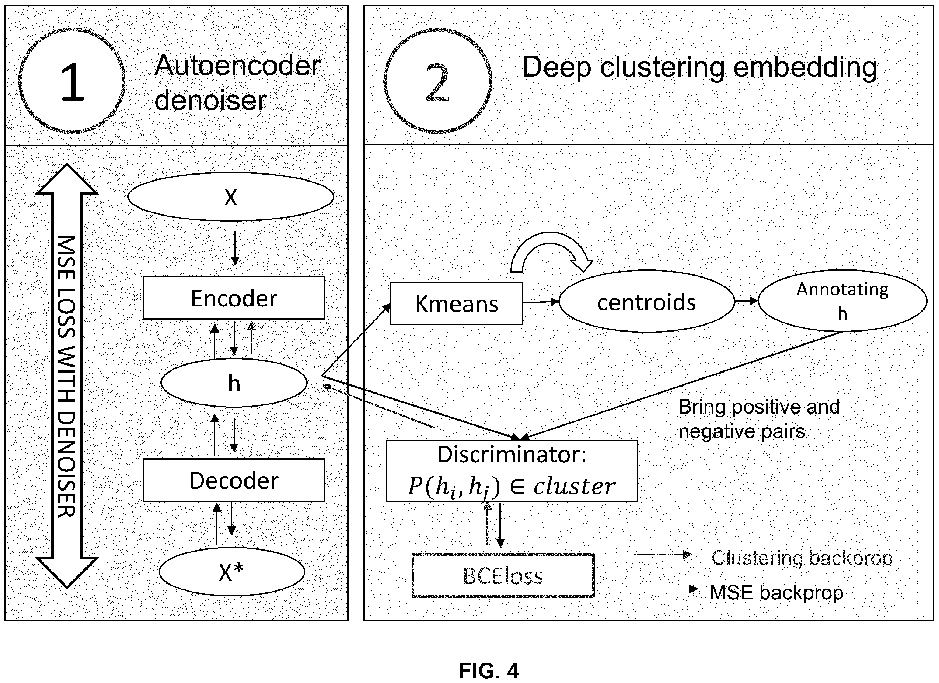

As explained, this is the basic functioning of any known autoencoder, as depicted by the **figure 4.** In this figure, X is a gene expression vector, h its projection into the embedding space, and X* its reconstructed approximation.

In the following step (a1) a clustering algorithm is applied to the projections into said embedding space of the training gene expression vectors of said batch so that each training gene expression vector of the batch is assigned to a cluster among a plurality of clusters. By "clustering", it is meant the task of grouping a set of objects in such a way that objects in the same group (called a cluster) are more similar (in some sense) to each other than to those in other groups (clusters). In other words, in the embedding space the various projections are attempted to be grouped.

Numerous clustering algorithm are known to the skilled person, in particular "centroid-model" algorithm such as k-means.

The k-means algorithm aims to partition n objects into k clusters in which each object belongs to the cluster with the nearest mean ("cluster centroid", i.e. the center of the cluster), serving as a prototype of the cluster. This results in a partitioning of the embedding space into Voronoi cells. k-means clustering minimizes within-cluster variances (squared Euclidean distances). Centroids coordinates can be conserved during training (with possibly a random initialization), and projection of training vectors into the embedding space can be annotated with the annotations (cell types) associated to the corresponding inputted training vectors (if the training database has already such ground truth annotations).

Note other algorithms such as "mixture model" can be used. Mixture model clustering fits a set of probabilistic models to the data. These models can be a variety of probability distributions but are most commonly Gaussian distributions. This clustering approach is called mixture modeling because multiple (a mixture of) probability distributions are fitted to the data.

At the end of step (a1), the different training gene expression vectors of a batch are clustered into the embedding space. The performance of the clustering, i.e. the quality of segregation of objects of one cluster from items from others clusters actually depends from the embedding space (the clustering algorithm is itself optimal). While the skilled person already knows how to train autoencoder to very effectively approximate their input, it is not yet known how to pass through an embedding space which is optimal for clustering.

The present method astutely uses to this end a "discriminator" neural network which engages with the autoencoder (in particular its encoder block) in other to assess the clustering quality, as represented by figure 4. More precisely, in a step (a2), for a plurality of pairs of training gene expression vectors of said batch (possibly randomly selected), it is predicted with said discriminator neural network whether they are assigned to the same cluster, and preferably whether they are identically annotated (if there are cluster annotations, see before). Note that in practice, the discriminator directly works on the projections of said pair, noted $(h_i, h_j)$ in figure 4.

In order words, for a pair the discriminator attempts to determine if the two vectors belong or not to the same cluster (what is called a "positive" or a "negative" pair). If clusters are well segregated, the discriminator will have no trouble correctly distinguish positive pairs from negative pairs. Else, the embedding space shall be improved.

It is to be understood that the pair of the encoder block of the autoencoder and the discriminator can be seen as some kind of Generative Adversarial Network (GAN).

In a final step (a3) the autoencoder (and the discriminator) are actually trained by modifying parameters of the autoencoder-type neural network and the discriminator neural network so as to minimize a loss function, in a known fashion (in particular backpropagation). Note that there may be a warmup training with only the autoencoder.

By loss function, or "cost" function, it is meant a function whose value tends towards zero when the behavior of the networks to be trained is optimal.

Here, the loss function comprises at least two terms, preferably summed, each term being a potentially standalone loss (i.e. a positive value that should tends toward 0).

The first term representative of a reconstruction error of the autoencoder-type neural network (it can be referred to as "reconstruction loss"), as a function of the training gene expression vectors and the reconstructed approximations of said training gene expression vectors. The first term is well known and is the (whole) loss function general used for training autoencoders. It is typically the Mean-Square error (or another error) between the training gene expression vectors and the reconstructed approximations of said training gene expression vectors.

More originally, the second term is representative of a cluster segregation error of the autoencoder-type neural network (it can be referred to as "clustering loss"), as a function of the predictions of the discriminator. Indeed, the first term only assesses the ability of the autoencoder to reconstruct its input, but it does not assess the choice or the embedding space.

By contrast, this second term is a custom supplemental loss that allows the training to improve simultaneously the performances of the encoder and the quality of the embedding space. By "cluster error", it is meant an error depicting the lack of segregation of the clustering and the poor separation (even the merging) of two clusters.

The second term uses the predictions of the discriminator: preferably, said second term tends toward 0 if the discriminator neural network correctly predicts whether a pair of training gene expression vector are assigned to the same cluster (i.e. it predicted that vectors of a positive pairs belong to the same cluster, or that vectors of a negative pairs belong to different clusters), and tends toward 1 if the discriminator neural network incorrectly predicts whether a pair of training gene expression vectors are assigned to the same cluster i.e. (it predicted that vectors of a negative pairs belong to the same cluster, or that vectors of a positive pairs belong to different clusters).

To this end, the discriminator neural network may be chosen to output a predicted probability score (between 0 and 1) that a pair of training gene expression vectors are assigned to the same cluster: if the score is 0, the discriminator is 100% sure that the pair is a negative pair, if the score is 1, the discriminator is 100% sure that the pair is a positive pair, and if the score is 0.5, the discriminator cannot tell if the pair is a negative or a positive pair.

Said second term can thus be computed based on the difference between said predicted probability score and a ground truth equal to 1 if the pair of training gene expression vectors are actually assigned to the same cluster, and equal to 0 if the pair of training gene expression vectors are actually not assigned to the same cluster. Mathematically, if G is the ground truth and p the predicted probability score, said value is

$$\begin{cases} p \; if \; G = 0 \\ 1 - p \; is \; G = 1 \end{cases}$$ and is equal to zero only in case of perfect prediction.

In a preferred embodiment, said second term of the loss is typically the Binary Cross Entropy, i.e. the log of said difference, expressed as -G*log(p)-(1-G)*log(1-p)).

The more the segregation is clean, the more the discriminator output correct predictions with high confidence (i.e. values close to 0 or 1), and hence the more the second term is close to 0.

As explained, the first and second are typically summed, preferably according to the formula *loss = MSE + λ\*BCE,* wherein MSE is the first term (Mean-Square Error), *BCE* is the second term (Binary Cross Entropy), and $\lambda$ a scalar with $0 < \lambda \leq 1$, preferably $0.05 \leq \lambda \leq 0.5$, more preferably $0.05 \leq \lambda \leq 0.2$, and for instance $\lambda = 0.1$.

Note that said training method is preferably repeated for a plurality of batches of training gene expression vectors from said training database for improving the autoencoder and the discriminator.

*Analysis method*

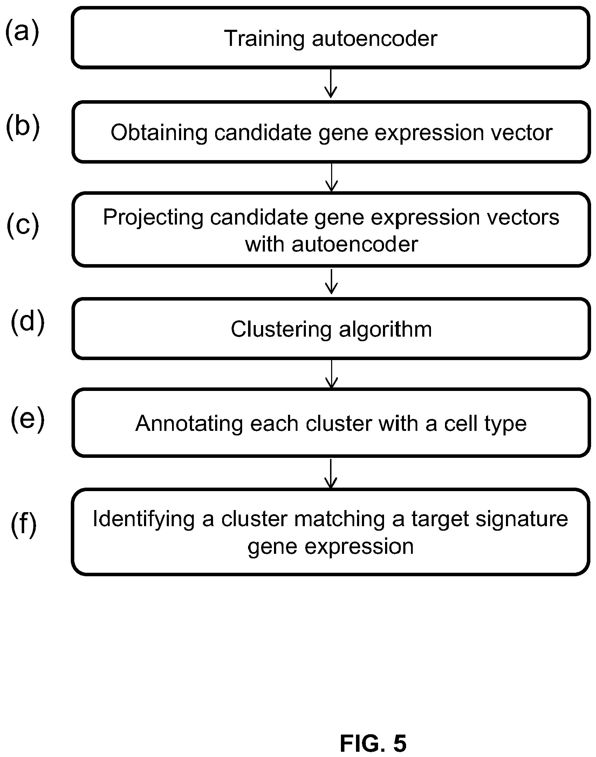

In a second aspect, with reference to **figure 5,** there is proposed a method for transcriptomic analysis of a biological sample implemented by the data processing means 11b of the second server 1b. In other words, the method according to the second aspect analyzes a set of candidate gene expression vectors ("expression matrix") each representing at least a part of a transcriptome of a cell of said biological sample.

Said set of candidate gene expression vectors to be analyzed can be received from the client equipment 10.

At an optional first step (a), the training of an autoencoder-type neural network is performed by the first server 1a (from a base of training gene expression vectors). Preferably, said training is consistent with the method according to the first aspect, i.e. using the discriminator.

It is to be understand that either the first and the second server 1a, 1b are the same equipment, or step (a) comprise the transmission of the trained autoencoder parameters and weights from the first server 1a to the second server 1b, for storage in the memory 12b. Note that the discriminator is not required for performing said method according to the second aspect.

Note that it is sufficient that said autoencoder-type neural network is obtainable by the method according to the first, i.e. its embedding space allows clear segregation of clusters, but it could be taken "off the shelf".

At a second step (b), the processing means 11b of the second server 1b obtains from the client equipment 2, for a plurality of individual cells of said biological sample, the candidate gene expression vector representing at least a part of a transcriptome of the cell. Note that "candidate" vector is here intended by contrast with "training" vector. Preferably, the same genes are involved for the training and candidate vectors. As already explained, this step (b) can be performed by any means known to the skilled person.

In a step (c), for each candidate gene expression vector of the set of said candidate gene expression vectors, the candidate gene expression vector is projected into said embedding space of the given reduced dimension with respect to the dimension of the gene expression vector, with the encoder block of the autoencoder-type neural network.

Then, said clustering algorithm is applied to the projections into said embedding space of the candidate gene expression vectors of said set so that each candidate gene expression vector of the set is assigned to a cluster among a plurality of clusters. Because of the proper training (in particular the discriminator and the second term of the loss), the clusters will be well segregated.

Note that the embedding space is still of a dimension too high for visualization (typically 64). In order to further reduce this dimension, in particular to 2 or 3, a dimensional reduction algorithm such as t-SNE (t-distributed stochastic neighbor embedding) can be used. This is a statistical method for visualizing high-dimensional data by giving each datapoint a location in a two or three-dimensional map.

As illustrated by figure 1b the result is very clear. We see the round shape of clusters due to the clustering loss.

The analysis method preferably comprises a further step (e) of annotating each cluster with a cell type as a function of the most differentially expressed genes in candidate gene expression vectors of said cluster, for instance by excluding the genes referred to as "housekeeping genes" which are the least differentially expressed genes in candidate gene expression vectors of said clusters. This is also well known to the skilled person.

The method preferably even comprises a further step (f) of identifying a cluster matching a target signature gene expression in candidate gene expression vectors of said cluster.

As explained, the objective is to allow, for each cell, to determine the expression of identified targets and biomarkers using an improved treatment algorithm. In other word to know in which cell type the target or the biomarker is expressed, depending on the disease, the disorder, or the inflammation, for example a cancer indication. Targeting may indeed refer to upregulating or downregulating the activity of a target gene or gene product.

For example, this invention improves selection of targets and biomarkers in cancer indications.

The improvement of the algorithm allows:

- ranking of cancer indications with the highest level/frequency of biomarkers or targets on cells of interest.
- checking the identified biomarkers or targets in a specific cancer indication.

A broad set of datasets aggregated (at patient or indication scale) can be evaluated with fine-tuning from the analysis of the relevant cell type separation (with higher resolution, limited background noise, and good separation between the clusters).

*Computer program product*

In a third and fourth aspect, the invention concerns a computer program product comprising code instructions to execute a method (particularly on the data processing means 11a, 11b of the first or second server 1a, 1b) according to the first aspect of the invention for training an autoencoder-type neural network for transcriptomic analysis of a biological sample, or a method according to the second aspect of the invention for transcriptomic analysis of a biological sample, and storage means readable by computer equipment (memory of the first or second server 1a, 1b) provided with this computer program product.

**Claims**

1. A method for training an autoencoder-type neural network for transcriptomic analysis of a biological sample, the method being **characterized in that** it comprises the implementation, by a data processor (11a) of a first server (1a), for at least a batch of training gene expression vectors from a database, each training gene expression vector representing at least a part of a transcriptome of a cell, of steps of:

    (a0) for each training gene expression vector of said batch,

        - projecting the training gene expression vector into an embedding space of a given reduced dimension with respect to the dimension of the gene expression vector, with an encoder block of the autoencoder-type neural network;
        - reconstructing an approximation of the training gene expression vector from its projection into said embedding space, with an encoder block of the autoencoder-type neural network;

    (a1) applying a clustering algorithm to the projections into said embedding space of the training gene expression vectors of said batch so that each training gene expression vector of the batch is assigned to a cluster among a plurality of clusters;
    (a2) for a plurality of pairs of training gene expression vectors of said batch, predicting with a discriminator neural network whether they are assigned to the same cluster;
    (a3) modifying parameters of the autoencoder-type neural network and the discriminator neural network so as to minimize a loss function comprising at least:

        - a first term representative of a reconstruction error of the autoencoder-type neural network, as a function of the training gene expression vectors and the reconstructed approximations of said training gene expression vectors;
- a second term representative of a cluster segregation error of the autoencoder-type neural network, as a function of the predictions of the discriminator.

2. A method according to claim 1, wherein said first term is the Mean-Square error between the training gene expression vectors and the reconstructed approximations of said training gene expression vectors.

3. A method according to any one of claims 1 and 2, wherein said second term tends toward 0 if the discriminator neural network correctly predicts whether a pair of training gene expression vector are assigned to the same cluster, and tends toward 1 if the discriminator neural network incorrectly predicts whether a pair of training gene expression vectors are assigned to the same cluster

4. A method according to claim 3, wherein the discriminator neural network outputs a predicted probability score that a pair of training gene expression vectors are assigned to the same cluster, said second term being computed as the Binary Cross Entropy between said predicted probability score and a ground truth equal to 1 if the pair of training gene expression vectors are actually assigned to the same cluster, and equal to 0 if the pair of training gene expression vectors are actually not assigned to the same cluster.

5. A method according to any one of claims 1 to 4, wherein said loss function is given by the formula $MSE + \lambda * BCE$, wherein MSE is the first term, $BCE$ is the second term, and $\lambda$ a scalar with $0 < \lambda \leq 1$.

6. A method according to any one of claims 1 to 5, wherein each of the encoder block and the decoder block comprises two sequences of a core layer and a non-linear activation layer, wherein the core layer is chosen among a fully connected layer, an attention layer, and a convolution layer.

7. A method according to claim 6, wherein both the first core layer of the encoder and the first core layer of the decoder comprise 256 nodes, and the second core layer of the encoder comprises 64 nodes, so that said embedding space is of dimension 64.

8. A method according to any one of claims 1 to 7, wherein the gene expression vectors are of dimension at least 10000, in particular at least 20000.

9. A method according to any one of claims 1 to 8, wherein said clustering algorithm is the k-means algorithm.

10. A method for transcriptomic analysis of a biological sample, the method being **characterized in that** it comprises the implementation, by a data processor (11b) of a second server (1b), of steps of:

(b) Obtaining from a client equipment (2), for a plurality of individual cells of said biological sample, a candidate gene expression vector representing at least a part of a transcriptome of the cell;
(c) for each candidate gene expression vector of the set of said candidate gene expression vectors, projecting the candidate gene expression vector into an embedding space of a given reduced dimension with respect to the dimension of the gene expression vector, with an encoder block of an autoencoder-type neural network obtainable by the method according to any one of claims 1 to 9;
(d) applying said clustering algorithm to the projections into said embedding space of the candidate gene expression vectors of said set so that each candidate gene expression vector of the set is assigned to a cluster among a plurality of clusters.

11. A method according to claim 10, comprising a previous step (a) of training said autoencoder-type neural network according to any one of claims 1 to 9.

12. A method according to any one of claims 10 and 11, comprising a further step (e) of annotating each cluster with a cell type as a function of the most differentially expressed genes in candidate gene expression vectors of said cluster.

13. A method according to any one of claims 10 to 12, comprising a further step (f) of identifying a cluster matching a target signature gene expression in candidate gene expression vectors of said cluster.

14. Computer program product comprising code instructions to execute a method according to one of claims 1 to 13 for training an autoencoder-type neural network, or for transcriptomic analysis of a biological sample, when said program is executed on a computer

15. A computer-readable medium, on which is stored a computer program product comprising code instructions for executing a method according to any one of claims 1 to 13 for training an autoencoder-type neural network, or for transcriptomic analysis of a biological sample.

**FIG. 1a**

**FIG. 1b**

**FIG. 2**

FIG. 3

FIG. 4

(a) Training autoencoder

(b) Obtaining candidate gene expression vector

(c) Projecting candidate gene expression vectors with autoencoder

(d) Clustering algorithm

(e) Annotating each cluster with a cell type

(f) Identifying a cluster matching a target signature gene expression

**FIG. 5**

Europäisches
Patentamt

European
Patent Office

Office européen
des brevets

## EUROPEAN SEARCH REPORT

Application Number

EP 22 30 5728

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WU YULUN ET AL: "AAE-SC: A scRNA-Seq Clustering Framework Based on Adversarial Autoencoder", IEEE ACCESS, IEEE, USA, vol. 8, 26 September 2020 (2020-09-26), pages 178962-178975, XP011812984, DOI: 10.1109/ACCESS.2020.3027481 [retrieved on 2020-10-06] * whole document, in particular abstract; page 178963, right column; paragraphs "AAE-SC framework" and "Training strategy and automation" on pages 178966-178967; Table 2; paragraph "implementation details" on page 178969; Figures * | 1-15 | INV. G16B20/00 G16B25/10 G16B40/30 |
| X | CN 111 785 329 A (NAT UNIV DEFENSE TECHNOLOGY PLA) 16 October 2020 (2020-10-16) * the whole document * | 1-15 | |
| X | LIN EUGENE ET AL: "A deep adversarial variational autoencoder model for dimensionality reduction in single-cell RNA sequencing analysis", BMC BIOINFORMATICS, BIOMED CENTRAL, LONDON, vol. 21, no. 1, 21 February 2020 (2020-02-21), XP021273407, DOI: 10.1186/S12859-020-3401-5 [retrieved on 2020-02-21] * whole document, in particular abstract and Methods; Figure 1 * | 1-15 | TECHNICAL FIELDS SEARCHED (IPC) G16B |

-/--

The present search report has been drawn up for all claims

| Place of search | Date of completion of the search | Examiner |
|---|---|---|
| Munich | 14 October 2022 | Vanmontfort, D |

CATEGORY OF CITED DOCUMENTS

X : particularly relevant if taken alone
Y : particularly relevant if combined with another
document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

page 1 of 2

Europäisches
Patentamt

European
Patent Office

Office européen
des brevets

## EUROPEAN SEARCH REPORT

Application Number

EP 22 30 5728

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | Flores Mario ET AL: "Deep learning tackles single-cell analysis – A survey of deep learning for scRNA-seq analysis Running title: Deep learning for single-cell RNA-seq analysis", , 1 January 2021 (2021-01-01), XP055970682, Retrieved from the Internet: URL:https://arxiv.org/ftp/arxiv/papers/2109/2109.12404.pdf [retrieved on 2022-10-12] * the whole document * ----- | 1-15 | |

TECHNICAL FIELDS
SEARCHED      (IPC)

The present search report has been drawn up for all claims

| Place of search | Date of completion of the search | Examiner |
|---|---|---|
| Munich | 14 October 2022 | Vanmontfort, D |

EPO FORM 1503 03.82 (P04C01)

page 2 of 2

## ANNEX TO THE EUROPEAN SEARCH REPORT
## ON EUROPEAN PATENT APPLICATION NO.

**EP 22 30 5728**

This annex lists the patent family members relating to the patent documents cited in the above-mentioned European search report.
The members are as contained in the European Patent Office EDP file on
The European Patent Office is in no way liable for these particulars which are merely given for the purpose of information.

**14-10-2022**

| Patent document cited in search report | Publication date | Patent family member(s) | Publication date |
|---|---|---|---|
| CN 111785329 A | 16-10-2020 | NONE | |

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **DONGQING SUN, ; JIN WANG ; YA HAN ; XIN DONG ; JUN GE ; RONGBIN ZHENG ; XIAOYING SHI ; BINBIN WANG ; ZIYI LI ; PENGFEI REN.** TISCH: a comprehensive web resource enabling interactive single-cell transcriptome visualization of tumor microenvironment. *Nucleic Acids Research,* 08 January 2021, vol. 49 (D1), D1420-D1430, https://doi.org/10.1093/nar/gkaa1020